# EUROPÄISCHE PATENTSCHRIFT

(11) **EP 2 181 374 B1**
(45) Veröffentlichungstag und Bekanntmachung des Hinweises auf die Patenterteilung: **07.09.2011**
(21) Anmeldenummer: 08774009.8
(22) Anmeldetag: 17.07.2008
(51) Int. Cl.: G05D 7/06

(54) **DOSIER- UND VERSORGUNGSSYSTEM FÜR VORRICHTUNGEN ZUM H2O2-STERILISIEREN VON PACKMITTELN SOWIE VORRICHTUNG MIT EINEM SOLCHEN DOSIER- UND VERSORGUNGSSYSTEM**
DOSING AND SUPPLY SYSTEM FOR DEVICES FOR H2O2 STERILIZATION OF PACKAGING MATERIALS AND DEVICE HAVING SUCH A DOSING AND SUPPLY SYSTEM
SYSTÈME DE DOSAGE ET D'ALIMENTATION POUR DISPOSITIFS DE STÉRILISATION D'EMBALLAGES AU H2O2 ET DISPOSITIF ÉQUIPÉ D'UN TEL SYSTÈME DE DOSAGE ET D'ALIMENTATION

(30) Priorität: 17.08.2007 DE 102007039010
(43) Veröffentlichungstag der Anmeldung: 05.05.2010
(73) Patentinhaber: KHS GmbH, 44143 Dortmund (DE)
(72) Erfinder: HEROLD, Thomas, 22926 Ahrensburg (DE); TOPF, Roland, 22177 Hamburg (DE)
(86) Internationale Anmeldenummer: PCT/EP2008/005828
(87) Internationale Veröffentlichungsnummer: WO 2009/024218

(56) Entgegenhaltungen:
- DE-A1- 3 637 798
- DE-A1- 4 036 950
- DE-A1- 19 956 186

## Beschreibung

Die Erfindung bezieht sich auf ein Dosler- und Versorgungssystem gemäß Oberbegriff Patentanspruch 1 sowie auf eine Vorrichtung zum H₂O₂-Sterilisieren von Packmitteln gemäß Oberbegriff Patentanspruch 14.

"H₂O₂-Sterilisation" im Sinne der Erfindung bedeutet eine Sterilisation von Packmitteln unter Verwendung von Wasserstoffperoxid (H₂O₂). Dabei kann das H₂O₂ sowohl In einem Sterilisationsmedium enthalten sein, als auch alleiniger Bestandteil eines Sterilisationsmediums sein. Nachstehend wird nicht zwischen beiden Möglichkeiten unterschieden, vielmehr wird nachfolgend der Begriff H₂O₂-Sterilisationsmedium verwendet.

"Packmittel" im Sinne der Erfindung sind insbesondere Flaschen oder dergleichen Behälter, einschließlich KEGs, Dosen, Tuben, aber auch andere Verpackungen, wie z.B. Weichverpackungen oder Beutel, die vor dem Einbringen von Produkten, z.B. Lebens- oder Genussmittel, wie beispielsweise Getränken, oder Arzneimittel sterilisiert werden müssen.

Verfahren und Vorrichtungen zum Sterilisieren von Packmitteln unter Verwendung eines H₂O₂-Sterilisationsmediums, welches Wasserstoffperoxid in Mischung mit heißer steriler Luft enthält und in Behandlungsköpfen der jeweiligen Vorrichtung zur H₂O₂-Sterilisation gebildet wird, sind bekannt Bei diesem Verfahren wird mit dem über den jeweiligen Behandlungskopf In das Packmittel eingebrachten heißen H₂O₂-Sterilisationsmedium an der Innenfläche des kühleren Packmittels durch Kondensation ein H₂O₂-Kondensationsfilm gebildet, der dann in einer darauffolgenden Aktivierungsphase durch Einbringen eines sterilen heißen gasund/oder dampfförmigen Aktivierungsmediums, beispielsweise durch Einbringen von heißer steriler Luft aktiviert wird, sodass durch Zerfall von H₂O₂ freie Sauerstoffradikale entstehen, die zur Sterilisation mit vorhandenen Kelmen und Verunreinigungen reagieren.

Um eine H₂O₂-Sterilisation mit hoher Qualität, d.h. mit hoher Entkeimungsrate Insbesondere auch mit hoher Zuverlässigkeit und auch reproduzierbar und auch nachweisbar durchzuführen und dabei den Verbrauch an Wasserstoffperoxid möglichst gering zu halten, ist eine genaue Dosierung der Wasserstoffperoxid-Zugabe bei der Bildung das H₂O₂-Sterilisationmediums an die verwendeten Behandlungskopfe notwendig.

Ein Dosier-Versorgungssystem mit den Merkmalen des Oberbegriffs des Patentanspruches 1 ist bekannt (DE 40 36 950 A1). Bei dem bekannten System erfolgt die H₂O₂-Sterilisation von Packmitteln mit einem einzigen Behandlungskopf, über den das an einer Zerstäuberdüse zerstäubte Wasserstoffperoxid in das jeweilige Packmittel eingebracht wird. Während der H₂O₂-Sterilisation wird das Wasserstoffperoxid mit Hilfe einer Pumpe in einem Wasserstoffperoxid-Kreislauf gefördert, der zusätzlich zu der Pumpe u.a. ein Vorratsbehälter sowie eine Entgasungseinrichtung enthält. Über eine Leitung, die an die Entgasungseinrichtung abzweigend angeschlossen ist und damit nicht Bestandteil des Wasserstoffperoxid-Kreislaufes während der Behandlung der Packmittel ist und außerdem einen Durchflussmesser sowie ein von diesem angesteuertes Ventil enthält, wird die der Entgasungseinrichtung entnommene Teilmenge des Wasserstoffperoxids der Zerstäuberdüse zugeführt.

Bekannt sind weiterhin ein Verfahren sowie eine Vorrichtung zum Sterilisieren von Verpackungsbehältern (DE 199 56 186 A1) mit einer Injektionsmaschine mit einem um die vertikale Maschinenachse umlaufend antreibbaren Drehtisch oder Rotor, an welchem u.a. mehrere Behandlungsköpfe für eine Behandlung mit einem Gemisch aus Wasserdampf und einem Desinfektionsmittel vorgesehen sind. Dieses enthält in wässriger Lösung als keimtötende Bestandteile Wasserstoffperoxid und Peressigsäure. Das Mischen des Wasserdampf und des Desinfektionsmittels erfolgt in dem jeweiligen Behandlungskopf. Ringleitungen oder dergleichen für einen Wasserstoffperoxid-Kreislauf sind nicht vorgesehen.

Aufgabe der Erfindung ist es, ein Dosier- und Versorgungssystem aufzuzeigen, weiches eine optimale Wasserstoffperoxid-Dosierung mit reproduzierbaren Ergebnissen ermöglicht. Zur Lösung dieser Aufgabe ist ein Dosier- und Versorgungssystem entsprechend dem Patentanspruch 1 ausgebildet. Eine Vorrichtung für die Durchführung der H₂O₂-Sterilisation ist Gegenstand des Patentanspruchs 14.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung In den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: in sehr vereinfachter Darstellung und in Draufsicht eine Maschine bzw. Vorrichtung zum H₂O₂-Sterilisieren von Packmitteln in Form von Flaschen;

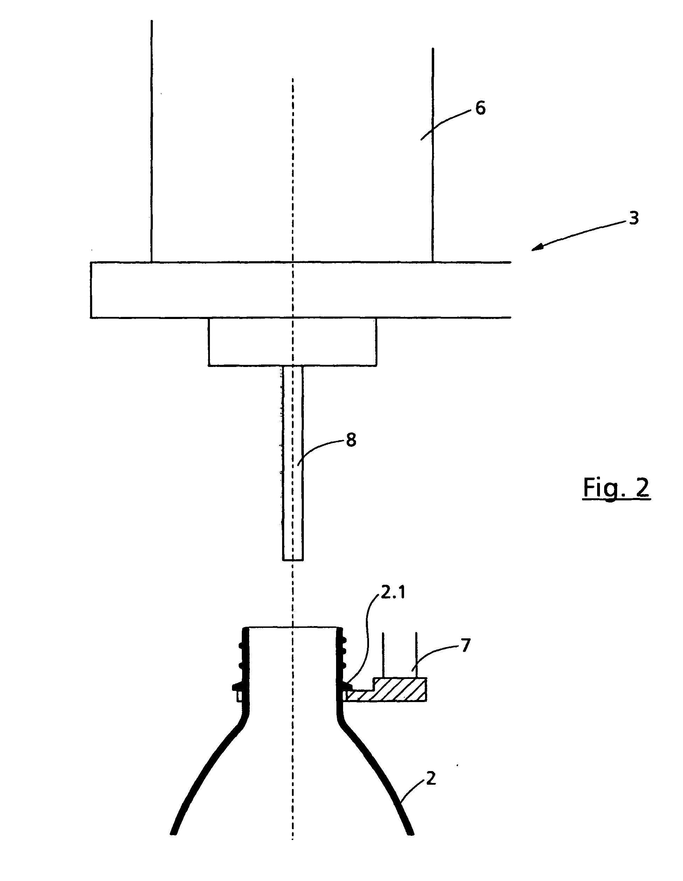

- Fig. 2: in vereinfachter Darstellung einen Behandlungskopf der Vorrichtung der Figur 1;
- Fig. 3: in Funktionsdarstellung ein System zur dosierten Wasserstoffperoxid- Versorgung mehrerer Behandlungsköpfe der Vorrichtung gemäß Figur 1;
- Flg. 4: eine Darstellung wie Figur 3 bei einer weiteren Ausführungsform des erfindungsgemäßen Systems.

In den Figuren ist 1 eine Vorrichtung zum Behandeln bzw. Sterilisieren von Flaschen mit Wasserstoffperoxid (H₂O₂). Die Vorrichtung besteht im Wesentlichen aus einem um eine vertikale Achse umlaufend angetriebenen Rotor 3, dem die zu behandelnden Flaschen 2 über einen Behältereinlauf 4 zugeführt werden und von dem die behandelten bzw. sterilisierten Flaschen 2 über einen Behälterauslauf 5 entnommen und einer weiteren Verwendung, beispielsweise einem Füller zum Füllen mit einem Füllgut zugeführt werden.

Am Umfang des Rotors 3 ist in gleichmäßigen Winkelabständen um die vertikale Maschinen- oder Rotorachse versetzt eine Vielzahl von Behandlungsköpfen 6 vorgesehen. Jedem Behandlungskopf ist am Rotor ein Flaschen-oder Behälterträger 7 zugeordnet, an dem die jeweilige Flasche 2 während der Behandlung unterhalb des Behandlungskopfes 6 gehalten ist, und zwar bei der dargestellten Ausführungsform die als PET-Flaschen ausgebildeten Flaschen 2 an einem flaschenseitigen Mündungsflansch hängend.

Die Behandlung, d.h. die Sterilisation der Flaschen 2 erfolgt unter Verwendung des H₂O₂-Sterilisationsmediums, welches in an sich bekannter Weise innerhalb einer in dem Behandlungskopf 6 gebildeten Mischkammer 6.1 durch Einsprühen von Wasserstoffperoxid, beispielsweise von 35-%igen Wasserstoffperoxid über wenigstens eine Mischdüse in sterile Luft zur Bildung eines Wasserstoffperoxid/Luft-Aerosols und durch anschließendes Erhitzen dieses Aerosols in einem Verdampfer 6.2 auf eine Temperatur von beispielsweise 145°C erzeugt wird. An der Mischkammer ist ein Anschluss 6.1.1 für die sterile Luft sowie ein weiterer Anschluss 6.1.2 für das Wasserstoffperoxid vorgesehen.

Für die Behandlung wird ein achsgleich mit der Achse der jeweiligen Flasche 2 angeordnetes Rohr 8 des Behandlungskopfes 6 in die Flasche 2 eingeführt und über dieses Rohr in einer Applikationsphase zunächst das heiße H₂O₂-Sterilisationsmedium in das innere der Flasche 2 eingebracht, und zwar derart, dass sich durch Kondensation an der Innenfläche der Flasche 2 ein H₂O₂-Kondensationsfilm bildet. Im Anschluss an diese Applikationsphase erfolgt in der Aktivierungsphase die Aktivierung des H₂O₂-Kondensationsfilmes durch Energieeintrag, und zwar z.B. durch Einleitung eines heißen, sterilen gas- und/oder dampfförmigen Mediums, beispielsweise durch Einleiten von heißer steriler Luft über das Rohr 8, so dass durch Zerfallreaktion oder Spaltung von H₂O₂ freie SauerstoffRadikale entstehen, die mit vorhandenen Keimen und/oder Verunreinigungen in der jeweiligen Flasche 2 reagieren und so deren Sterilisation bewirken.

Um einerseits den Verbrauch an Wasserstoffperoxid zu optimieren, andererseits aber eine Sterilisation mit hoher Qualität, d.h. mit hoher Entkeimungsrate reproduzierbar zu erreichen, ist eine genaue und insbesondere auch eine reproduzierbare Dosierung der über den Anschluss 6.1.2 der jeweiligen Mischkammer 6.1 zugeführten Menge an Wasserstoffperoxid unerlässlich.

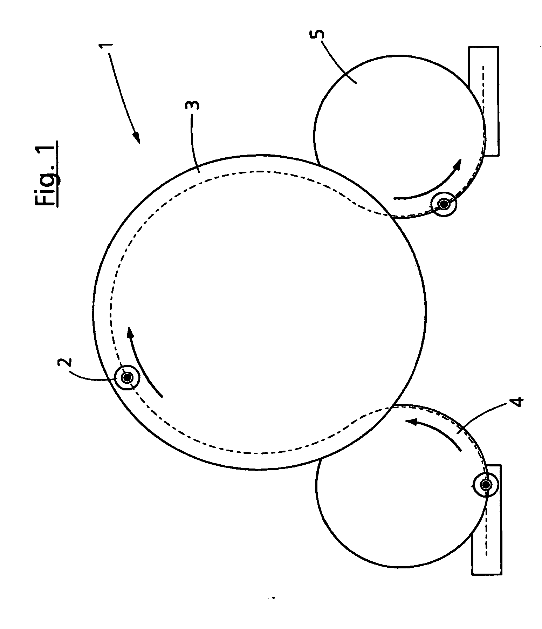

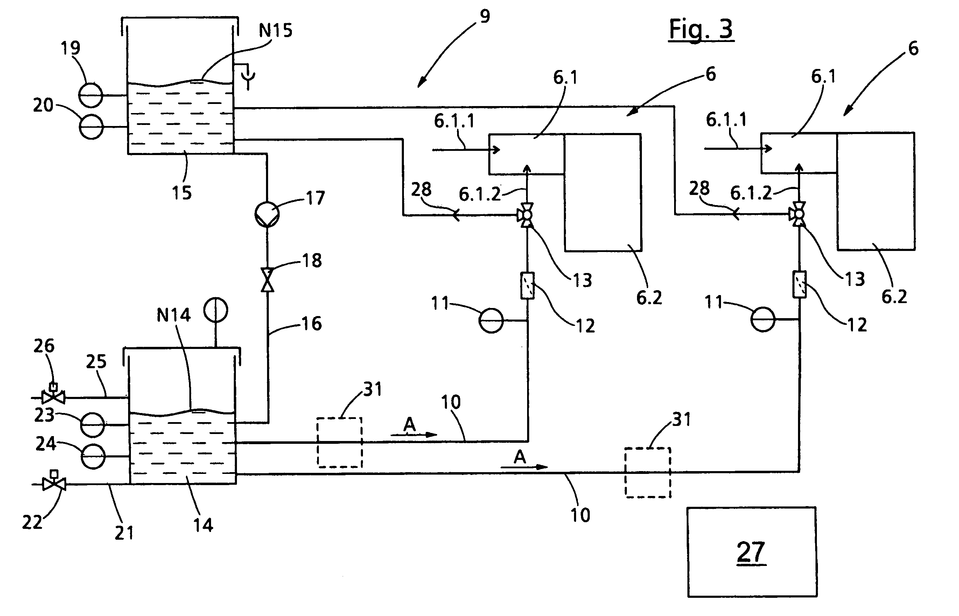

Die Figur 3 zeigt in vereinfachter Darstellung und als Blockdiagramm ein Versorgungs- und Dosiersystem zum Versorgen bzw. dosierten Zuführen von Wasserstoffperoxid an die einzelnen Behandlungsköpfe 6 der Vorrichtung der Figur 1, wobei in der Figur 3 der einfacheren Darstellung wegen nur zwei derartige Behandlungsköpfe 6 gezeigt sind.

Ein grundsätzliches Problem bei der dosierten Zuführung von Wasserstoffperoxid an die Behandlungsköpfe ist, dass das H₂O₂ als ein wenig stabiles Produkt bereits bei normaler Umgebungstemperatur ständig in Wasser und Sauerstoff zerfällt, wobei die Zerfallsrate insbesondere durch eventuelle plötzliche Druckänderungen und/oder Druckstöße in einem H₂O₂ führenden System noch unkontrolliert gesteigert wird.

Nach einer der Erfindung zugrunde liegenden Erkenntnis ist somit eine dosierte Zuführung von Wasserstoffperoxid aus einem Vorratsbehälter über dosierende Pumpen nicht ohne weiteres möglich, und zwar u.a. deswegen, weil sich durch den Zerfall des Wasserstoffperoxids in derartigen Pumpen, aber auch in Zuleitungen an bzw. von den Pumpen Sauerstoffblasen bilden, die eine genaue Dosierung nicht möglich machen.

Dieses Problem wird durch die Ausbildung des in der Figur 3 allgemein mit 9 bezeichneten Dosier- und Versorgungssystems gelöst. Dieses System sieht für jeden Behandlungskopf 6 einen Wasserstoffperoxid-Kreislauf vor, der jeweils von einer Ringleitung 10 gebildet ist, die in Richtung des Pfeils A während des Betriebs der Vorrichtung 1 ständig von Wasserstoffperoxid durchströmt wird und in der in Strömungsrichtung A aufeinander folgend ein Durchflussmesser 11, ein Filter 12 sowie ein Dosierventil 13 in Form eines 2/3-Wegeventils vorgesehen sind. Letzteres ist mit einem Eingang und einem der beiden Ausgänge in der Ringleitung 10 angeordnet ist und mit seinem zweiten Ausgang an den Anschluss 6.1.2 des jeweiligen Behandlungskopfs angeschlossen ist. Jede Ringleitung 10 erstreckt sich zwischen einem Vorrats- oder Versorgungsbehälter 14, der das Wasserstoffperoxid unter Druck (Dosierdruck) enthält und aus dem das Wasserstoffperoxid in die Ringleitungen 10 fließt, sowie einen Sammelbehälter 15 zum Sammeln des über die Ringleitungen 10 zurückfließenden Wasserstoffperoxid und zum Abscheiden von Sauerstoff an der Phasengrenzfläche bzw. Wasserstoffperoxidspiegel zwischen dem flüssigen Wasserstoffperoxid im Flüssigkeitsraum des Sammelbehälters 15 und dem oberhalb des Wasserstoffperoxidspiegels gebildeten Gasraum des Sammelbehälters 15. Der abgeschiedene Sauerstoff wird dann z.B. in die Atmosphäre abgeleitet oder einer weiteren Verwendung, beispielsweise einer Einrichtung zur Erzeugung von Ozon für Sterilisationszwecke zugeführt. Aus dem Sammelbehälter 15 wird das Wasserstoffperoxid über eine Verbindungsleitung 16 an den Vorrats- oder Versorgungsbehälter 14 zurückgeführt. In der Verbindungsleitung 16 ist hierfür eine Pumpe 17 in Serie mit einem Rückschlagventil 18 vorgesehen.

Der Druck im Sammelbehälter 15 ist niedriger als der Dosierdruck im Versorgungsbehälter 14 und entspricht beispielsweise Atmosphärendruck. Die

Pumpe 17 wird in Abhängigkeit von dem Ausgangssignal zweier Niveauschalter 19 und 20 gesteuert, sodass das Niveau N₁₅ des Flüssigkeitsspiegels des Wasserstoffperoxids im nur teilgefüllten Sammelbehälter 15 zwischen einem vorgegebenen Maximalwert und einem vorgegebenen Minimalwert gehalten wird. Der Versorgungsbehälter 14 ist über eine Versorgungsleitung 21 mit Steuerventil 22 an eine, Wasserstoffperoxid unter Druck bereitstellende, Quelle angeschlossen. Weiterhin sind am Vorrats- oder Versorgungsbehälter 14 zwei Niveauschalter 23 und 24 vorgesehen, über die Wasserstoffperoxid-Zufuhr an den ebenfalls nur teilgefüllten Versorgungsbehälter 14 durch entsprechende Ansteuerung des Steuerventils 22 derart geregelt wird, dass das Niveau N₁₄ des Flüssigkeitsspiegels des Wasserstoffperoxids in dem Versorgungsbehälter 14 sich stets zwischen einem maximalen und einem minimalen Wert befindet. Der oberhalb des Flüssigkeitsspiegels im Versorgungsbehälter 14 gebildete Gasraum ist über eine Versorgungsleitung 25 mit Steuerventils 26 mit einer Quelle verbunden, die sterile Luft unter Druck bereitstellt. In Abhängigkeit von dem Signal eines am Versorgungsbehälter 14 vorgesehenen Druckmessers 27 wird das Steuerventil 26 derart gesteuert, dass sich ein konstanter Dosierdruck im Versorgungsbehälter 14 ausbildet. Grundsätzlich besteht auch die Möglichkeit, zur Regelung des Druckes in dem Vorratsbehälter 14 das Ventil 26 als rückwärts entlüftendes Druckminderventil auszubilden.

Die Arbeitsweise des Dosier- und Versorgungssystems 9 lässt sich, wie folgt, beschreiben:

Die Dosierventile 13 sind beispielsweise so ausgebildet, dass sie im nichtaktivierten Zustand einen kontinuierlichen Wasserstoffperoxid-Strom durch die einzelnen Ringleitungen 10 von dem Versorgungsbehälter 14 in den Sammelbehälter 15 ermöglichen. Durch diesen Umlauf ist gewährleistet, dass sich in jeder Ringleitung 10, insbesondere auch in dem Leitungsabschnitt in Strömungsrichtung vor dem jeweiligen Dosierventil 13 ausschließlich oder nahezu ausschließlich nur Wasserstoffperoxid ohne oder im Wesentlichen ohne Sauerstoff- oder Gasblasen befindet, und dass aus dem Wasserstoffperoxid abgespaltener Sauerstoff an dem Flüssigkeitsspiegel bzw. an der Phasengrenzfläche im Sammelbehälter 15 abscheidet.

Zur dosierten Abgabe des Wasserstoffperoxids an den jeweiligen Behandlungskopf 6 bzw. an die dortige Mischkammer 6.1 wird das betreffende Dosierventil 13 vorzugsweise periodisch aktiviert und wieder deaktiviert, wobei beispielsweise im aktivierten Zustand mittels des Durchflussmessers 11 der Volumenstrom des Wasserstoffperoxids erfasst und in Abhängigkeit von einem entsprechenden, an die Steuereinrichtung 27 gelieferten Messsignals das Dosierventil 13 so gesteuert wird, dass die an dem jeweiligen Behandlungskopf 6 erforderliche Wasserstoffperoxid-Dosierung erreich wird, wobei bei dieser Dosierung bzw. bei diesem gesteuerten Öffnen und Schließen der Dosierventile 13 durch die Steuereinrichtung 27 selbstverständlich der in der Ringleitung 10 herrschende Dosierdruck ebenso berücksichtigt wird, wie u.a. auch der Volumenstrom der der jeweiligen Mischkammer 6.1 zufließenden sterilen Luft.

Um ein Abscheiden oder ein erhöhtes Abscheiden von Sauerstoff aus dem Wasserstoffperoxid zumindest in dem Teil der jeweiligen Ringleitung 10 vor dem Dosierventil 13 beim Aktivieren und Deaktivieren dieses Ventils zu vermeiden und um sicherzustellen, dass der Dosierdruck an den Dosierventilen 13 ansteht, ist in jeder Ringleitung 10 in Strömungsrichtung auf das Dosierventil 13 folgend oder am entsprechenden Ausgang des Dosierventils 13 eine Drossel 28 vorgesehen, die so gewählt bzw. eingestellt ist, dass sich für den Wasserstoffperoxid-Strom an beiden Ausgängen jedes Dosierventils 13 der selbe oder annähernd der selbe Strömungswiderstand ergibt, also auch der Volumenstrom des Wasserstoffperoxids im Abschnitt der Ringleitung 10 vor dem jeweiligen Dosierventil unabhängig von dem Zustand dieses Ventils ist und sich daher auch keine Druckstöße oder abrupte Druckänderungen in der jeweiligen Ringleitung 10 vor dem Dosierventil 13 ergeben.

Eine Besonderheit des Dosier- und Versorgungssystems 9 besteht auch darin, dass die Förderung des Wasserstoffperoxids durch die Ringleitungen 10 und insbesondere auch durch die Dosierventile 13 ausschließlich durch den Dosier- bzw. Kopfdruck im Versorgungsbehälter 14 erfolgt, in dem eigentlichen Umlauf des Wasserstoffperoxids zwischen den beiden Behältern 14 und 15 also keine Pumpen vorgesehen sind. Durch diese Vorgehensweise wird eine besonders gleichmäßige Strömung erzielt, welche frei von den üblicherweise durch Pumpen verursachten Druckschwankungen ist.

Die Verbindung zwischen dem Dosierventil 13 und dem Anschluss 6.1.2 bzw. der Dosierdüse der Mischkammer 6.1 des zugehörigen Behandlungskopfes 6 ist möglichst kurz gehalten, um die Verweildauer des Wasserstoffperoxids innerhalb dieser Verbindung und damit die Abspaltung von Sauerstoff aus dem Wasserstoffperoxid möglichst gering zu halten.

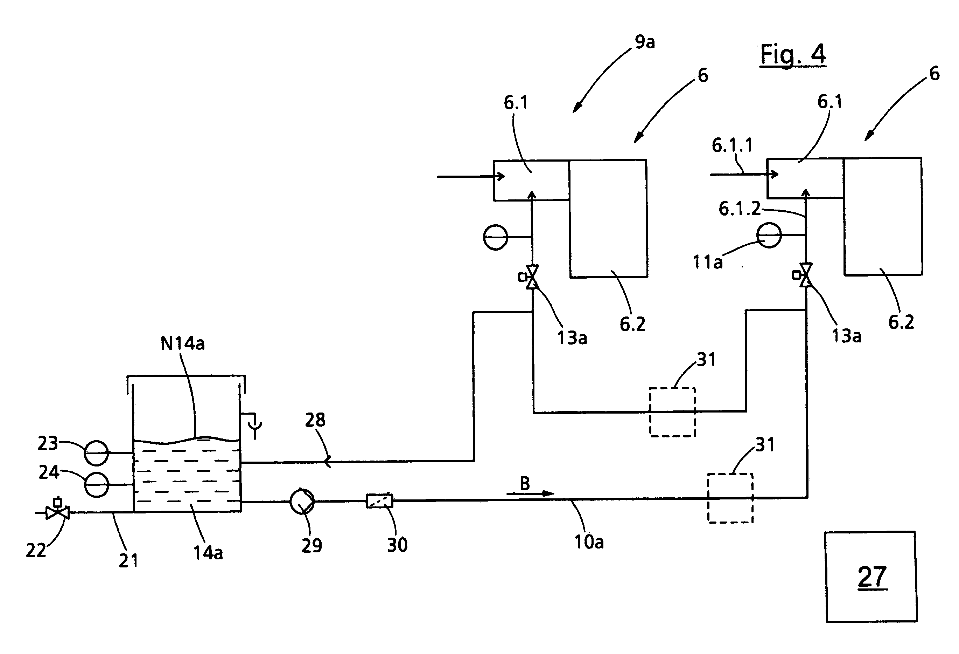

Die Figur 4 zeigt als weitere Ausführungsform ein Dosiersystem 9a, welches ebenfalls zur Verwendung bei der Vorrichtung 1 geeignet ist, wobei auch in der Figur 4 wiederum der einfacheren Darstellung wegen nur zwei Behandlungsköpfe 6 dargestellt sind.

Ein wesentlicher Unterschied zu dem Dosier- und Versorgungssystem 9 besteht bereits darin, dass das Dosier- und Versorgungssystem 9a lediglich eine einzige Ringleitung 10a für sämtliche Behandlungsköpfe 6 aufweist, und dass diese Ringleitung 10a über eine Pumpe 29, beispielsweise Kreiselpumpe, und über ein in Serie mit dem Pumpenausgang liegendes Filter 30 an den Versorgungsbehälter 14a angeschlossen ist, der das Wasserstoffperoxid im drucklosen Zustand, d.h. bei Atmosphärendruck enthält. Über eine Drossel 28a ist die Ringleitung 10a an den Vorratsbehälter 14a zurückgeführt, sodass bei eingeschalteter Pumpe 29 sich ein Wasserstoffperoxid-Kreislauf aus dem Vorratsbehälter 14a durch die Pumpe 29, das Filter 30 und die Drossel 28a zurück in den Vorratsbehälter 14a in Richtung des Pfeils B ergibt. In diesem Wasserstoffperoxid-Kreislauf oder -Umlauf ist in Strömungsrichtung nach der Pumpe 29 bzw. nach dem Filter 30 und vor der Drossel 29a für jeden Behandlungskopf 6 ein Dosierventil 13a vorgesehen, welches gesteuert durch die Steuereinrichtung 27 geöffnet und geschlossen wird, also im Gegensatz zu den Dosierventilen 13 nicht zwischen zwei Ausgängen umschaltet. In der Verbindungsleitung zwischen dem jeweiligen Dosierventil 13a und der Mischkammer 6.1 bzw. der dortigen Mischdüse ist bei dieser Ausführungsform jeweils ein Durchflussmesser 11a für jeden Behandlungskopf 6 gesondert vorgesehen, welcher bei aktiviertem, d.h. geöffneten Dosierventil 13a die dem jeweiligen Behandlungskopf 6 zufließende Menge an Wasserstoffperoxid erfasst und ein entsprechendes Messsignal zur Steuerung der Dosierung bzw. des zugehörigen Dosierventils 13a an die Steuereinrichtung 27 liefert. Auch bei dieser Ausführungsform ist die jeweilige Verbindung zwischen dem Dosierventil 13a und der Mischkammer 6.1 des zugehörigen Behandlungskopfes 6 möglichst kurz, um die Verweildauer des Wasserstoffperoxids in dieser Verbindung und damit das Abspalten von Sauerstoff gering zu halten.

Der Versorgungsbehälter 14a ist wiederum über die Versorgungsleitung 21 mit dem Steuerventil 22 an eine Versorgungsquelle für das Wasserstoffperoxid angeschlossen. Zur Regelung des Niveaus N₁₄a des Flüssigkeitsspiegels im Versorgungsbehälter 14a bzw. zur Steuerung des Steuerventils 22 sind am Vorratsbehälter 14a wiederum die beiden Niveauschalter 23 und 24 vorgesehen.

Die Arbeitsweise des Dosier- und Versorgungssystems 9a lässt sich, wie folgt, beschreiben:

Bei eingeschalteter Pumpe 29 wird ein ständiger Wasserstoffperoxid-Umlauf durch die Ringleitung 10a aus dem Vorratsbehälter 14a und wieder zurück in diesen Vorratsbehälter erreicht und dabei insbesondere auch erreicht, dass sich aus dem Wasserstoffperoxid abscheidender Sauerstoff im Versorgungsbehälter 14a an dem dortigen Flüssigkeitsspiegel bzw. an der dortigen Phasengrenzfläche abscheidet und beispielsweise in die Atmosphäre abgeleitet oder einer weiteren Verwendung, beispielsweise einer Einrichtung zur Erzeugung von Ozon für Sterilisationszwecke zugeführt wird. Das in der Ringleitung 10a umlaufende Wasserstoffperoxid ist somit frei oder zumindest im Wesentlichen frei von Gas- oder Sauerstoffblasen. Durch ein gesteuertes Öffnen und Schließen der Dosierventile 13a erfolgt das Dosieren des Wasserstoffperoxids, wobei bei dieser Dosierung bzw. bei diesem gesteuerten Öffnen und Schließen der Dosierventile 13a von der Steuereinrichtung 27 selbstverständlich wiederum der in der Ringleitung 10a herrschende und von der Pumpe 29 erzeugte Dosierdruck ebenso berücksichtigt wird, wie u.a. auch der Volumenstrom der der jeweiligen Mischkammer 6.1 zufließenden sterilen Luft.

Der Querschnitt der Ringleitung 10a ist ausreichend groß gewählt, sodass Druckverluste und/oder Druckschwankungen und/oder Änderungen des Volumenstromes des Wasserstoffperoxids bedingt durch das periodische, aber gesteuerte Öffnen und schließen der Dosierventile 13a vernachlässigbar sind und insbesondere auch an jedem Dosierventil 13a jeweils der identische, konstante bzw. geregelte Dosierdruck anliegt. Hierdurch ist dann das Dosiervolumen ausschließlich von der Schalt- bzw. Öffnungszeit des jeweiligen Dosierventils 13a abhängig und auch ein durch Druckverlust und/oder Druckschwankungen und/oder Änderungen des Volumenstromes bedingtes Abscheiden von Sauerstoff aus dem Wasserstoffperoxid ist verhindert. Die Drossel 28a ist wiederum so eingestellt, dass der durch diese Drossel erzeugte Strömungswiderstand gleich oder im Wesentlichen gleich demjenigen Strömungswiderstand ist, den die über die Dosierventile 13a angesteuerten Mischkammern 6.1 in Summe bzw. in Serie für den Wasserstoffperoxid-Strom bilden.

Die Erfindung wurde voranstehend an Ausführungsbeispielen beschrieben. Es versteht sich, dass Änderungen sowie Abwandlungen möglich sind, ohne dass dadurch der der Erfindung zugrunde liegende Erfindungsgedanke verlassen wird.

Die Erfindung wurde vorstehend im Zusammenhang mit der H₂O₂-Sterilisation von Flaschen 2 erläutert. Die Erfindung, insbesondere auch die beschriebenen Dosiersysteme 9 und 9a sind aber für die H₂O₂-Sterilisation von anderen Packmitteln oder Verpackungsmittel, wie beispielsweise Schraubverschlüssen ebenfalls geeignet.

Vorstehend wurde weiterhin davon ausgegangen, dass bei Verwendung des mit dem Dosierdruck beaufschlagten Versorgungsbehälters 14 für jeden Behandlungskopf 6 eine eigene Ringleitung 10 mit einem als 2/3-Wege-Ventil ausgebildeten Dosierventil 13 vorgesehen ist. Grundsätzlich besteht aber auch bei Verwendung eines druckbeaufschlagten Versorgungsbehälters 14 die Möglichkeit, für sämtliche Behandlungsköpfe 6 oder aber für eine Gruppe von Behandlungsköpfen 6 eine gemeinsame Ringleitung entsprechend der Ringleitung 10a vorzusehen, und zwar mit Steuerventilen, die zumindest während des Dosierens lediglich zwischen einem Schließzustand und einem geöffnetem Zustand steuerbar sind.

Weiterhin ist es grundsätzlich auch möglich, bei einem drucklosen Versorgungsbehälter 14a für jeden Behandlungskopf 6 oder aber für eine Gruppe von Behandlungsköpfen 6 jeweils eine eigenständige Ringleitung mit Dosierventil vorzusehen, wobei der Dosierdruck dann mit einer in jeder Ringleitung angeordneten Pumpe oder aber mit einer Pumpe erzeugt wird, die für sämtliche oder aber mehrere Ringleitungen gemeinsam ist.

Weiterhin kann es zweckmäßig sein, in der jeweiligen, jedem Behandlungskopf eigenständig zugeordneten Ringleitung 10 oder aber in einer für sämtliche Behandlungsköpfe oder aber einer Gruppe von Behandlungsköpfen gemeinsamen Ringleitung 10a zumindest einen zusätzlichen Gasabscheider zum Entfernen von aus dem Wasserstoffperoxid abgespaltetem Sauerstoff vorzusehen, und zwar bevorzugt jeweils in Strömungsrichtung des Wasserstoffperoxids vor dem betreffenden Dosierventil, wie dies in den Figuren 3 und 4 mit unterbrochenen Linien bei 31 angedeutet ist.

Weiterhin kann es zweckmäßig sein, die jedem einzelnen Behandlungskopf 6 zugeführte Menge an Wasserstoffperoxid gezielt zu überwachen und zu regeln. Dazu ist vorgesehen, die von jedem einzelnen Durchflussmesser 11a gelieferten Informationen innerhalb einer Steuereinheit auszuwerten und zur Ansteuerung des zugeordneten Dosierventils 13a zu verwenden.

### Bezugszeichenliste

- 1: Vorrichtung
- 2: Flasche
- 3: Rotor
- 4: Einlauf
- 5: Auslauf
- 6: Behandlungskopf
- 6.1: Mischkammer
- 6.2: Verdampfer
- 6.1.1: Anschluss für Sterilluft mit Mischkopfdüse
- 6.1.2: Anschluss für H₂O₂ mit Mischkopfdüse
- 7: Flaschen- bzw. Behälterträger
- 8: Rohr
- 9, 9a: Dosier- und Versorgungssystem
- 10, 10a: Ringleitung
- 11, 11a: Durchflussmesser
- 12: Filter
- 13, 13a: Dosierventil
- 14, 14a: Vorrats- oder Versorgungsbehälter für H₂O₂
- 15: Sammelbehälter
- 16: Leitung
- 17: Pumpe
- 18: Rückschlagventil
- 19, 20: Niveauschalter
- 21: Versorgungsleitung
- 22: Steuerventil
- 23, 24: Niveauschalter
- 25: Versorgungsleitung
- 26: Steuerventil
- 27: elektronische Steuereinrichtung
- 28, 28a: Drossel
- 29: Pumpe
- 30: Filter
- 31: Gasabscheider
- A: H₂O₂-Strömungsrichtung im Ringkanal 10
- B: H₂O₂-Strömungsrichtung im Ringkanal 10a
- N₁₄, N₁₅: Niveau

## Patentansprüche

1. Dosier- und Versorgungssystem zum dosierten Zuführen von Wasserstoffperoxid an einer Vorrichtung zur H₂O₂-Sterilisation von Packmitteln (2), mit wenigstens einem das Wasserstoffperoxid bereitstellenden Vorratsbehälter (14, 14a) und mit einer steuerbaren Verbindung zwischen dem wenigstens einem Vorratsbehälter (14, 14a) und einem Behandlungskopf (6), wobei die steuerbare Verbindung wenigstens eine Ringleitung (10, 10a) gebildet ist, die zusammen mit dem wenigstens einen Vorratsbehälter (14, 14a) einen während der H₂O₂-Sterilisation wirksamen Kreis- oder Umlauf für das Wasserstoffperoxid bildet, und wobei der jeweilige Behandlungskopf (6) über wenigstens ein Dosierventil (13, 13a) an die wenigstens eine Ringleitung (10, 10a) angeschlossen Ist,
**dadurch gekennzeichnet,**
**dass** eine Vielzahl von Behandlungsköpfe (6) mit jeweils einem Dosienrentil (13, 13a) vorgesehen ist, und dass die Dosierventile (13, 13a) unmittelbar in oder an der Ringleitung (10, 10a) für den Wasserstoffperoxid-Kreislauf während des Dosierens angeordnet sind.

2. Dosier- und Versorgungssystem nach Anspruch 1, **dadurch gekennzeichnet, dass** für sämtliche Behandlungsköpfe (6) oder eine Gruppe von mehreren Behandlungsköpfen (6) eine gemeinsame Ringleitung (10a) vorgesehen ist, und dass der Querschnitt der Ringleitung (10a) so groß gewählt ist, sodass Druckverluste und/oder Druckschwankungen und/oder Änderungen des Volumenstromes des Wasserstoffperoxids In der Ringleitung (10a) bedingt durch das periodische Öffnen und Schließen der Dosierventile (13a) während des Dosierens an jedem Dosierventil (13a) jeweils ein identischer, konstanter und geregelter Dosierdruck anliegt.

3. Dosier- und Versorgungssystem nach Anspruch 1, **dadurch gekennzeichnet, dass** für jeden Behandlungskopf (6) oder eine Gruppe von Behandlungsköpfen (6) jeweils eine Ringleitung (10) vorgesehen ist, und dass die Dosierventile (13) zumindest teilweise Umschaltventlle sind, die während des Dosierens zwischen einem den Volumenstrom durch die Ringleitung (10) ermöglichenden und einem den Volumenstrom an den betreffenden Behandlungskopf (6) leitenden Zustand steuerbar sind.

4. Dosier- und Versorgungssystem nach Anspruch 3, **dadurch gekennzeichnet, dass** in beiden Zuständen des jeweiligen Dosierventils (13) der Strömungswiderstand für das dieses Ventil durchströmende Wasserstoffperoxid identisch oder im Wesentlichen identisch ist.

5. Dosier- und Versorgungssystem nach einem der vorhergehenden Ansprüche,
**dadurch gekennzeichnet, dass** über das jeweilige Dosierventil (13, 13a) eine Mischkammer (6.1) bzw, wenigstens eine In dieser Mischkammer angeordnete Mischdüse des jeweiligen Behandlungskopfes (6) an die wenigstens eine Ringleitung (10, 10a) angeschlossen ist, mit der (Mischdüse) ein Mischen des Wasserstoffperoxids mit einem der Mischkammer (6.1) ebenfalls zugeführten gas- und/oder dampfförmigen Träger, beispielsweise mit Luft erfolgt.

6. Dosier- und Versorgungssystem nach einem der vorhergehenden Ansprüche,
**dadurch gekennzeichnet, dass** in dem von der Ringleitung (10) gebildeten Kreislauf zusätzlich zu dem wenigstens einen Vorratsbehälter (14) zumindest ein Sammelbehälter zum Sammeln des über die Ringleitung (10) rückgeführten Wasserstoffperoxids vorgesehen ist.

7. Dosier- und Versorgungssystem nach Anspruch 6, **dadurch gekennzeichnet, dass** der wenigstens eine Sammelbehälter (15) über eine Verbindungsleitung (16) mit dem Vorratsbehälter (14) in Verbindung steht.

8. Dosier- und Versorgungssystem nach einem der vorhergehenden Ansprüche,
**dadurch gekennzeichnet, dass** zur Erzeugung eines erforderlichen Dosierdruckes der Versorgungsbehälter (14) mit einem geregelten Druck beaufschlagbar ist.

9. Dosier- und Versorgungssystem nach Anspruch 8, **dadurch gekennzeichnet, dass** der Druck im Sammelbehälter (15) kleiner ist als der an dem jeweiligen Dosierventil (13, 13a) anstehende Dosierdruck, und dass in der Verbindungsleitung zwischen dem Sammelbehälter (15) und dem Versorgungsbehälter eine Pumpe (17) vorgesehen ist.

10. Dosier- und Versorgungssystem nach einem der vorhergehenden Ansprüche,
**dadurch gekennzeichnet, dass** zur Erzeugung des an den Dosierventilen (13) anstehenden Dosierdruckes wenigstens eine Pumpe (29) vorgesehen Ist, die Bestandteil der wenigstens einen Ringleitung (10a) Ist.

11. Dosier- und Versorgungssystem nach einem der vorhergehenden Ansprüche,
**dadurch gekennzeichnet, dass** die Ringleitung (10a) für den Wasserstoffperoxid-Kreislauf während des Dosierens beidendig an den wenigstens einen Versorgungsbehälter (14a) angeschlossen ist.

12. Dosier- und Versorgungssystem nach einem der vorhergehenden Ansprüche, **dadurch gekennzeichnet, dass** in dem Wasserstoffperoxid-Kreislauf Mittel zum austragen von gasförmigen Zersetzungsprodukten des Wasserstoffperoxids vorgesehen sind, und dass die Mittel zum Austragen der gasförmigen Zersetzungsprodukte des Wasserstoffperoxids von der Phasengrenzfläche zwischen einem Flüssigkeitsraum und einem Gasraum in einem Gehäuse oder Behälter, beispielsweise in dem wenigstens einen Versorgungsbehälter (14, 14a) und/oder in dem wenigstens einen Sammelbehälter (15) gebildet sind.

13. Dosier- und Versorgungssystem nach einem der Ansprüche 3-12. **dadurch gekennzeichnet, dass** der wenigstens eine Vorratsbehälter (14) und/oder der wenigstens eine Sammelbehälter (15) für sämtliche Ringleitungen (10) gemeinsam vorgesehen ist.

14. Vorrichtung zur H₂O₂-Sterilisation von Packmitteln (2), mit mehreren Behandlungsköpfen (6) zum Einbringen jeweils eines erhitzten, aus Wasserstoffperoxid und einem dampf- und/oder gasförmigen Träger bestehenden H₂O₂-Sterilisationsmediums in das zu sterilisierende Packmittel (2) und zum anschließenden Aktivieren des H₂O₂-Sterilisationsmediums, sowie mit einem Dosier- und Versorgungssystem (9, 9a) zum dosierten Zuführen des Wasserstoffperoxids an die Behandlungsköpfe (6), wobei das Dosier- und Versorgungssystem (9, 9a) nach einem der vorhergehenden Ansprüche ausgebildet ist.

15. Vorrichtung nach Anspruch 14, **dadurch gekennzeichnet, dass** die Behandlungsköpfe (6) am Umfang eines um eine vertikale Achse umlaufend angetriebenen Rotors (3) vorgesehen sind.

## Claims

1. A dosing and supply system for the dosed supply of hydrogen peroxide to a device for H₂O₂ sterilization of packaging materials (2), having at least one supply container (14, 14a) providing the hydrogen peroxide and having a controllable connection between the at least one supply container (14, 14a) and a treatment head (6), the controllable connection being formed by at least one ring line (10, 10a) that, along with the at least one supply container (14, 14a), forms a circuit or cycle for the hydrogen peroxide effective during the H₂O₂ sterilization, and the respective treatment head (6) being connected via at least one dosing valve (13, 13a) to the at least one ring line (10, 10a), **characterized in that**
a plurality of treatment heads (6) each comprising a dosing valve (13, 13a) is provided and that the dosing valves (13, 13a) are disposed directly in or at the ring line (10, 10a) for the hydrogen peroxide circuit during the metering procedure.

2. Dosing and supply system according to claim 1, **characterized in that** a common ring line (10a) is provided for all treatment heads (6) or a group of a plurality of treatment heads (6), and that the cross-section of the ring line (10a) is selected to be so large that pressure losses and/or pressure fluctuations and/or changes of the volume flow of the hydrogen peroxide in the ring line (10a) due to the periodic opening and closing of the dosing valves (13a) during the metering procedure while a respective identical, constant and controlled dosing pressure is applied to each dosing valve (13a).

3. Dosing and supply system according to claim 1, **characterized in that** for each treatment head (6) or a group of treatment heads (6) a respective ring line (10) is provided and that the dosing valves (13) at least in part are switching valves which can be controlled during the metering procedure between a state enabling the volume flow through the ring line (10) and a state conducting the volume flow to the relevant treatment head (6).

4. Dosing and supply system according to claim 3, **characterized in that** in both states of the respective dosing valve (13) the flow resistance for the hydrogen peroxide flowing through said valve is identical or substantially identical.

5. Dosing and supply system according to any one of the preceding claims, **characterized in that** a mixing chamber (6.1) or at least a mixing nozzle disposed in this mixing chamber of the respective treatment head (6) is connected to the at least one ring line (10, 10a) via the respective dosing valve (13, 13a) and a mixing of the hydrogen peroxide with a gaseous or vaporous carrier also supplied to the mixing chamber (6.1), for example with air, is performed using the mixing valve.

6. Dosing and supply system according to any one of the preceding claims, **characterized in that** in the circuit formed by the ring line (10) at least one collecting container is provided additionally to the at least one supply container (14) for collecting the hydrogen peroxide returned via the ring line (10).

7. Dosing and supply system according to claim 6, **characterized in that** the at least one collecting container (15) is connected to the supply container (14) via a connecting line (18).

8. Dosing and supply system according to any one of the preceding claims, **characterized in that** for producing a required dosing pressure the supply container (14) can be pressurized with controlled pressure.

9. Dosing and supply system according to claim 8, **characterized in that** the pressure in the collecting container (15) is less than the dosing pressure applied to the respective dosing valve (13, 13a) and that a pump (17) is provided in the connecting line between the collecting container (15) and the supply container.

10. Dosing and supply system according to any one of the preceding claims, **characterized in that** for producing the dosing pressure applied to the dosing valves (13) at least one pump (29) is provided that is a component of the at least one ring line (10a).

11. Dosing and supply system according to any one of the preceding claims, **characterized in that** the ring line (10a) for the hydrogen peroxide circuit is connected at both ends to the at least one supply container (14a) during dosing.

12. Dosing and supply system according to any one of the preceding claims, **characterized in that** means for discharging gaseous decomposition products of the hydrogen peroxide are provided in the hydrogen peroxide circuit, and that the means for discharging the gaseous decomposition products of the hydrogen peroxide are formed by the phase interface between one fluid chamber and a gas chamber in a housing or container, for example in the at least one supply container (14, 14a) and/or in the at least one collecting container (15).

13. Dosing and supply system according to any one of claims 3 to 12, **characterized in that** the at least one supply container (14) and/or the at least one collecting container (15) is commonly provided for all ring lines (10).

14. Device for H₂O₂ sterilization of packaging materials (2) comprising a plurality of treatment heads (6) for respectively introducing a heated H₂O₂ sterilization medium consisting of hydrogen peroxide and a vaporous and/or gaseous carrier into the packaging material (2) to be sterilized, and for subsequently activating the H₂O₂ sterilization medium, and a dosing and supply system (9, 9a) for the dosed supply of the hydrogen peroxide to the treatment heads (6), the dosing and supply system (9, 9a) being configured according to any one of the preceding claims.

15. Device according to claim 14, **characterized in that** the treatment heads (6) are provided on the periphery of a rotor (3) rotatingly driven about a vertical axis.

## Revendications

1. Système de dosage et d'alimentation pour l'apport dosé de peroxyde d'hydrogène à un dispositif de stérilisation par H₂O₂ d'emballages (2) comportant au moins un réservoir de stockage (14, 14a) mettant à disposition le peroxyde d'hydrogène et un raccordement pouvant être commandé entre au moins le réservoir de stockage (14, 14a) et une tête de traitement (6), dans lequel le raccordement pouvant être commandé est formé d'au moins d'une conduite annulaire (10, 10a) qui, conjointement avec au moins le réservoir de stockage (14, 14a), forme un circuit ou une boucle actif/ve pour le peroxyde d'hydrogène pendant la stérilisation par H₂O₂, et dans lequel la tête de traitement respective (6) est raccordée par au moins une valve de dosage (13, 13a) à au moins la conduite annulaire (10, 10a),
**caractérisé en ce**
**qu'**une multiplicité de têtes de traitement (6) comportant respectivement une valve de dosage (13, 13a) est prévue, et en ce que les valves de dosage (13, 13a) sont disposées immédiatement dans ou sur la conduite annulaire (10, 10a) pour le circuit de peroxyde d'hydrogène pendant le dosage.

2. Système de dosage et d'alimentation selon la revendication 1, **caractérisé en ce que** pour toutes les têtes de traitement (6) ou un groupe de plusieurs têtes de traitement (6), une conduite annulaire commune (10a) est prévue, et **en ce que** la section de la conduite annulaire (10a) est choisie de sorte que pendant le dosage s'applique sur chaque valve de dosage (13a) respectivement, une pression de dosage identique, constante et régulée de sorte que des pertes de pression et/ou des variations de pressions et/ou des modifications du courant volumique du peroxyde d'hydrogène dans la conduite annulaire (10a) dues à l'ouverture et à la fermeture périodique des valves de dosage (13a) soient évitées.

3. Système de dosage et d'alimentation selon la revendication 1, **caractérisé en ce que**, pour chaque tête de traitement (6) ou groupe de têtes de traitement (6), respectivement une conduite annulaire (10) est prévue et **en ce que** les valves de dosage (13) sont au moins en partie des valves de commutation qui pendant le dosage entre un état permettant au courant volumique de passer par la conduite annulaire (10) et un état conduisant le courant volumique à la tête de traitement concernée (6), peuvent être commandées.

4. Système de dosage et d'alimentation selon la revendication 3, **caractérisé en ce que**, dans les deux états de la valve de dosage respective (13), la résistance au courant du peroxyde d'hydrogène traversant cette valve est identique ou essentiellement identique.

5. Système de dosage et d'alimentation selon l'une des revendications précédentes, **caractérisé en ce que** par la valve de dosage respective (13, 13a), une chambre de mélange (6.1) ou au moins une buse de mélange disposée dans cette chambre de mélange de la tête de traitement respective (6) est raccordée à au moins la conduite annulaire (10, 10a) avec laquelle (la buse de mélange), un mélange du peroxyde d'hydrogène est réalisé avec un véhicule sous forme de gaz et/ou de vapeur acheminé également à la chambre de mélange (6.1), par exemple avec de l'air.

6. Système de dosage et d'alimentation selon l'une des revendications précédentes, **caractérisé en ce que**, dans le circuit formé par la conduite annulaire (10) est prévu en plus d'au moins un réservoir de stockage (14) au moins un collecteur pour collecter le peroxyde d'hydrogène réacheminé par la conduite annulaire (10).

7. Système de dosage et d'alimentation selon la revendication 6, **caractérisé en ce qu'**au moins un collecteur (15) est raccordé par une conduite de raccordement (16) au réservoir de stockage (14).

8. Système de dosage et d'alimentation selon l'une des revendications précédentes, **caractérisé en ce que**, pour générer une pression de dosage nécessaire, le réservoir de stockage (14) est alimenté par une pression régulée.

9. Système de dosage et d'alimentation selon la revendication 8, **caractérisé en ce que** la pression dans le collecteur (15) est inférieure à la pression de dosage présente sur la valve de dosage respective (13, 13a), et **en ce que** dans la conduite de raccordement entre le collecteur (15) et le réservoir de stockage d'alimentation, une pompe (17) est prévue.

10. Système de dosage et d'alimentation selon l'une des revendications précédentes, **caractérisé en ce que**, pour générer la pression de dosage présente au niveau de la valve de dosage, au moins une pompe (29) est prévue, qui fait partie d'au moins la conduite annulaire (10a).

11. Système de dosage et d'alimentation selon l'une des revendications précédentes, **caractérisé en ce que** la conduite annulaire (10a) pour le circuit de peroxyde d'hydrogène pendant le dosage est raccordée des deux côtés à au moins un réservoir de stockage (14a).

12. Système de dosage et d'alimentation selon l'une des revendications précédentes, **caractérisé en ce que**, dans le circuit de peroxyde d'hydrogène, des moyens d'extraction des produits de décomposition sous forme gazeuse du peroxyde d'hydrogène sont prévus, et **en ce que** les moyens d'extraction des produits de décomposition sous forme gazeuse du peroxyde d'hydrogène sont formés par l'interphase entre un espace de liquide et un espace de gaz dans un logement ou dans un réservoir de stockage, par exemple dans au moins le réservoir de stockage (14, 14a) et/ou dans au moins le collecteur (15).

13. Système de dosage et d'alimentation selon l'une des revendications 3 à 12, **caractérisé en ce qu'**au moins un réservoir de stockage (14) et/ou au moins un collecteur (15) est prévu conjointement pour toutes les conduites annulaires (10).

14. Dispositif de stérilisation d'emballages (2) par H₂O₂, comportant plusieurs têtes de traitement (6) pour insérer respectivement un milieu de stérilisation par H₂O₂ consistant en peroxyde d'hydrogène chauffé et en un véhicule sous forme de vapeur et/ou de gaz dans l'emballage à stériliser (2) et pour ensuite activer le milieu de stérilisation par H₂O₂ et un système de dosage et d'alimentation (9, 9a) pour l'apport dosé de peroxyde d'hydrogène aux têtes de traitement (6), le système de dosage et d'alimentation (9, 9a) étant constitué selon l'une des revendications précédentes.

15. Dispositif selon la revendication 14, **caractérisé en ce que** les têtes de traitement (6) sont prévues sur le pourtour d'un rotor actionné circulairement autour d'un axe vertical.
